# EUROPÄISCHE PATENTSCHRIFT

(11) **EP 4 096 731 B1**
(45) Veröffentlichungstag und Bekanntmachung des Hinweises auf die Patenterteilung: **29.07.2026**
(21) Anmeldenummer: 21703158.2
(22) Anmeldetag: 25.01.2021
(51) Int. Cl.: A61L 27/30, A61K 6/20, A61K 6/818, A61K 6/824, C23C 14/00

(54) **WEISSE, BAKTERIENRESISTENTE, BIOKOMPATIBLE, HAFTFESTE BESCHICHTUNG FÜR IN HART- UND WEICHGEWEBE INTEGRIERTE IMPLANTATE, SCHRAUBEN UND PLATTEN UND HERSTELLUNGSVERFAHREN**
WHITE, BACTERIA-RESISTANT, BIOCOMPATIBLE, ADHERENT COATING FOR IMPLANTS, SCREWS AND PLATES INTEGRATED IN HARD AND SOFT TISSUE AND PRODUCTION METHOD
REVÊTEMENT ADHÉRENT BLANC, RÉSISTANT AUX BACTÉRIES, BIOCOMPATIBLE, POUR IMPLANTS, VIS ET PLAQUES INTÉGRÉS DANS DES TISSUS DURS ET MOUS ET PROCÉDÉ DE PRODUCTION

(30) Priorität: 27.01.2020 DE 102020101882
(43) Veröffentlichungstag der Anmeldung: 07.12.2022
(73) Patentinhaber: Implantissimo GmbH, 86825 Bad Wörishofen (DE)
(72) Erfinder: REPENNING, Detlev, 21465 Reinbek (DE)
(74) Vertreter: Meissner Bolte Partnerschaft mbB
(86) Internationale Anmeldenummer: PCT/EP2021/051602
(87) Internationale Veröffentlichungsnummer: WO 2021/151832

(56) Entgegenhaltungen:
- WO-A1-2007/128285
- WO-A1-2008/009474
- WO-A1-2008/056323
- DE-A1- 102014 011 972
- DATABASE WPI Week 201309, Derwent World Patents Index; AN 2012-H48149, XP002802827
- DATABASE WPI Week 201806, Derwent World Patents Index; AN 2017-79228P, XP002802829

## Beschreibung

Die vorliegende Erfindung bezieht sich auf ein Zahnimplantat mit einer weißen, bakterienresistenten, biokompatiblen, haftfesten Beschichtung, das in Hart- und Weichgewebe integrierbar ist, sowie auf ein zugehöriges Herstellungsverfahren. Beschichtungen, die insbesondere Anforderungen hinsichtlich bakterieller Resistenzen, bezüglich Biokompatibilitätsbedingungen und in Bezug auf chemische Resistenzen erfüllen, sind vielfach beschrieben Beispielhaft können die folgenden Veröffentlichungen genannt werden: R. Thull, Elektrochemische Prüfung von (Ti,Nb)ON beschichteten Legierungen, Biomedizinische Technik Bd. 36(9), 214ff, 1991; W.W. Plitz, Reib- und Verschleißuntersuchungen von (Ti,Nb)ON Schichten, Labor Biomechanik, München, 1994; R. Thull, K. Taubner, E. J. Kahle, biologische und tierexperimentelle Untersuchungen (Ti,Zr)O2 und (Ti,Nb)ON, Biomedizinische Technik, Bd. 37, 7-8, 1992; R. Thull, K. Taubner, E. J. Kahle, Modell zur immunologischen Prüfung von Biomaterialien, Biomedizinische Technik, Bd. 37, 162-169, 1992; D. Repenning, Materialempfindlichkeit bei Titan-Implantaten - Teil 1, Zahnheilkunde Management ZMK, 2010; M. Stelzel e. a., Verhalten verschiedener Titanoberflächen bei oraler Exposition - eine In-vivo-Studie, Philips Universität Marburg, Abt. Parodontologie 2003; D. Repenning, Oberflächenbeschichtung auf Implantaten, ossäre Integration, 53-61, Springer Verlag; Betz, Reuther, 5-jährige klinische Studie enossaler Implantate unter besonderer Berücksichtigung des periimplantären Gewebes, Deutsche Zeitschrift für Mund- und Kieferchirurgie, 1995.

All diesen Schichten haftet der Nachteil an, dass sie nicht das ästhetische Kriterium einer weißen Oberfläche erfüllen. Hierbei ist im vorliegenden Kontext unter "weiß" ein Farbbereich zwischen leicht grau über leicht rosa eingefärbt bis hin zu rein weiß zu verstehen, der in der jeweiligen organischen Umgebung als weiß wahrgenommen wird.

Besonders für beschichtete Zahnimplantate aus Titan besteht der Wunsch, ästhetisch weiße Oberflächen zur Verfügung zu stellen. Dieses gilt gleichermaßen für den enossalen Teil des Implantats als auch für das Abutement. Gleichzeitig unterliegen die Oberflächen den oben genannten Anforderungen zur Biokompatibilität (ELISA, Thull), sowie den Anforderungen eines effektiven Korrosionsschutzes gegen den Austritt von Ionen aus dem Substratmaterial und einer Bakterienresistenz, insbesondere gegen pathogene, anaerobe Bakterien. Nicht zuletzt muss eine topographisch optimal eingestellte Oberfläche eine sichere Osseointegration gewährleisten.

Die bislang im Verkehr befindlichen Beschichtungen weisen Farberscheinungen von goldfarben (Nitride), über dunkelblau (keramische Titan-Zirkonoxid Schichten) bis hin zu schwarz auf (DLC= diamond like carbon). Bekannte weißgraue Beschichtungen stellen Hydroxylapatitbeschichtungen (HA-Beschichtungen) dar, die der besseren Knochenintegration dienen. Diese HA-Beschichtungen werden jedoch zeitlich im Gewebe resorbiert. Sie werden zudem auch nur auf das enossale Teil eines Implantates appliziert und nicht auf das transgingivale Implantatteil.

Bekannt geworden sind auch weiße bzw. weißähnliche Titandioxidbeschichtungen, die durch elektrolytische Verfahren erzeugt werden. Allerdings erfüllen Titandioxide nicht die oben genannten Anforderungen zur Bakterienresistenz (siehe M. Stelzel e. a., Verhalten verschiedener Titanoberflächen bei oraler Exposition - eine In-vivo-Studie, Philips Universität Marburg, Abt. Parodontologie 2003) und nur in geringem Ausmaße die zur Biokompatibilität (siehe R. Thull, K. Taubner, E. J. Kahle, biologische und tierexperimentelle Untersuchungen (Ti,Zr)O2 und (Ti,Nb)ON, Biomedizinische Technik, Bd. 37, 7-8, 1992). Weitere bekannte Ansätze zur Beschichtung von Implantatkörpern basieren auf Sol-Gel-Verfahren und Spritzverfahren. Zu letzteren gehören das Plasmaspritzverfahren (APS), das Kaltspritzverfahren (CGS), das Hochgeschwindigkeitsflammspritzen (HVOF), das Lichtbogenspritzen (AS), das Pulverflammspritzen (PFS), und nicht zuletzt das Drahtflammspritzen. All diese Schichten leiden unter dem Nachteil, dass sie für die medizinische Anwendung nicht genügend haftfest zu applizieren sind oder/und mit ausreichender Präzision auf die feingliedrigen Implantatkörper aufzubringen sind.

Weiße Zahnimplantate werden derzeit aus Zirkondioxid-Vollkeramiken bereitgestellt. Zur Stabilisierung gegen Bruch sind die Keramiken mit Yttriumoxid dotiert. Keramische Implantate haben besonders den Nachtteil, dass ihre Oberflächen topographisch nicht optimal für eine sichere Osseointegration einstellbar sind und dass sie aufgrund der Bruchgefahr eine besondere und erhöhte Herausforderung an die Operation und Langzeitstabilität (bzw. Frühverluste im ersten Jahr) stellen. Zudem sind die Zusammensetzungen der Keramiken aus materialtechnisch systematischen Gründen nicht frei variierbar und ermöglichen so nicht die adäquate Anpassung des Materials an das biologische Milieu.

In der Patentliteratur bildet den nächstliegenden Stand der Technik die RO 127 411 A2. Weiteren relevanten Stand der Technik stellen die Patentdokumente WO 2008/056323 A1, DE 10 2014 011972 A1, WO 2008/009474 A1, WO 2007/128285 A1 und die CN 206 630 703 U dar.

Vor dem Hintergrund der obigen Ausführungen ist es Aufgabe der Erfindung, die genannten Nachteile aus dem Stand der Technik zu überwinden und eine Beschichtung für Implantate anzugeben, die sowohl ästhetische Kriterien hinsichtlich der Oberflächenfarbe erfüllen als auch eine gute Biokompatibilität, Langzeitstabilität und Bakterienresistenz aufweisen.

Diese Aufgabe wird durch ein Implantat mit den Merkmalen von Anspruch 1 gelöst. Die Aufgabe wird ferner durch ein Verfahren zur Herstellung eines Implantats mit den Merkmalen von Anspruch 1 gelöst.

Vorteilhafte Weiterbildungen ergeben sich aus den Unteransprüchen.

Ein wesentlicher Gedanke der Erfindung besteht darin, die Beschichtung mehrschichtig aus Gradientenschichten aufzubauen, wobei der Sauerstoffgehalt in den Gradientenschichten variiert. Vorzugsweise ist die unterste, auf dem Element aufgebrachte Gradientenschicht als metallische Haftvermittlungsschicht ausgebildet. Unter einer metallischen Haftvermittlungsschicht wird im Rahmen der vorliegenden Erfindung eine Gradientenschicht verstanden, die aus einem
Metall besteht und im Wesentlichen sauerstofffrei ist. Dadurch wird eine optimale Haftung auf dem Element erreicht. Die auf der metallischen Haftvermittlungsschicht ausgebildeten Gradientenschichten weisen vorzugsweise einen Sauerstoffgehalt auf, der von der untersten, auf dem Element aufgebrachten Gradientenschicht zu der äußersten Gradientenschicht auf die volle Stöchiometrie anwächst, derart, dass die äußerste Gradientenschicht eine Metalloxidschicht mit voller Stöchiometrie ist.

Erfindungsgemäß weisen die Gradientenschichten Tantal und/oder Niob auf. Diese Metalle zeichnen sich durch eine hohe biochemische Stabilität aus. Die äußerste Gradientenschicht ist titanfrei und weist eine Mischphase aus 20 Mol-% Nb₂O₃ und 80 Mol-% Ta₂O₅ auf.

Durch die graduelle Erhöhung des Sauerstoffgehalts über die Gradientenschichten wird die Haftung zwischen den Gradientenschichten verbessert. Sowohl die metallische Haftvermittlungsschicht als auch die Gradientenschichten mit Sauerstoffgehalt unterhalb der vollen Stöchiometrie der äußersten Metalloxid-Gradientenschicht weisen eine Färbung auf, die in den Gradientenschichten mit Sauerstoffgehalt durch Defekte in der Kristallstruktur hervorgerufen werden, an denen sich elektronische Zwischenzustände ausbilden, die zu einer charakteristischen Absorption und damit einer Färbung des Materials führen. Die äußerste Gradientenschicht mit voller Stöchiometrie ist kristallin und weist im Wesentlichen keine Defekte in der Struktur auf, die eine Färbung bewirken. Dadurch wird einerseits eine Beschichtung hergestellt, die sich aufgrund des sich graduell verändernden Sauerstoffgehalts von der metallischen Haftvermittlungsschicht zu der äußersten Metalloxid-Gradientenschicht durch ausgezeichnete Hafteigenschaften auszeichnet und gleichzeitig durch das Vorhandensein der äußersten Metalloxid-Gradientenschicht eine weiße Färbung aufweist.

Die Bandlücke der äußersten Metalloxid-Gradientenschicht ist größer als 3,1 eV, so dass die äußerste Schicht keine elektromagnetischen Wellen im sichtbaren Bereich absorbiert und damit weiß erscheint.

Zudem stellen Materialien, insbesondere keramische Systeme, mit Bandlücken von mehr als 3,1eV Werkstoffe mit hohem elektrischen Widerstand (R >> 1000 Ωcm⁻²) dar und tragen dazu bei, die Reaktion zwischen biologischem Gewebe und einem aus dem Material gebildeten Implantat durch Elektronenaustausch zu vermeiden.

Die erfindungsgemäß mit der Beschichtung erzielte Bakterienresistenz, Biokompatibilität und Haftfestigkeit ist für sämtliche Elemente von Vorteil, die im Zusammenhang mit einer Implantation eingesetzt werden. Daher ist die erfindungsgemäße Beschichtung für sämtliche Implantatkomponenten anwendbar. Es sei an dieser Stelle daher nochmals klarstellend darauf hingewiesen, dass sich die erfindungsgemäße Beschichtung für sämtliche Elemente und deren Oberflächen eignet, die in Hart- und Weichgewebe integrierbar sind oder mit solchen Elementen verbindbar sind, beispielsweise für sämtliche Oberflächen von Implantaten, Abutments und Verbindungselementen wie Schrauben, einschließlich deren gesamten Außenoberflächen sowie Innenoberflächen einschließlich Gewindeabschnitten.

Vorzugsweise weist mindestens eine der Gradientenschichten mit Sauerstoffgehalt, insbesondere mindestens die äußerste Metalloxid-Gradientenschicht, Korngrößen von 5 nm oder größer auf. Die nanokristalline Struktur mit Korngrößen im Submikrometerbereich trägt zur erhöhten Bruchzähigkeit der Schichten bei. Die Bestimmung der Korngrößen erfolgt mittels Röntgendiffraktometrie.

Gemäß dem erfindungsgemäßen Verfahren erfolgt die Aufbringung der Beschichtung vorzugsweise mittels eines PVD (physical vapour deposition)-Verfahrens. PVD-Verfahren werden typischerweise für die Beschichtung von medizinischen Implantaten und Instrumenten eingesetzt. Ihr Vorteil liegt in der hohen Variabilität, chemische Verbindungen einzustellen, seien es rein metallische, oxidische, nitridische, karbidische oder komplexere Zusammensetzungen.

Erfindungsgemäß werden oxidische, mindestens nanokristalline Schichten als Gradientenschichten mittels PVD Verfahren hergestellt, wobei die Bandlücke E_{g} zumindest der äußersten Gradientenschicht größer als 3,1eV ist. Die Haftung der Schichten wird mittels Gradientenschichten dadurch erreicht, dass zunächst eine metallische Haftvermittlungsschicht auf dem Element aufgebracht wird und im Folgenden der Sauerstoffgehalt der Schichten auf die volle Stöchiometrie eingestellt wird, vorzugsweise innerhalb von weniger als 500 nm. Wesentlich ist, dass die Gradientenschichten insoweit defektfrei eingestellt sind, dass sich keine Elektronenzustände einstellen, die entweder zur Verminderung der Bandlücke führen oder als Absorptionsszentren für längerwelliges Licht dienen.

Ein besonderer Nachteil der PVD-Verfahren, nämlich, dass die Abscheidungsbedingungen außerhalb des thermodynamischen Gleichgewichts erfolgen, wird dadurch behoben, dass die Beschichtung bei erhöhter Temperatur oberhalb T=300°C erfolgt und/oder dass die Schichten unter Sauerstoffatmosphäre nachträglich ausgeheilt werden. Der zusätzlich besondere Anspruch bezüglich der Bakterien-Resistenz wird bevorzugt über die mehrphasige Einstellung der Schichten erzielt. Mehrphasige Schichten variieren an der Oberfläche den pzzp (point of zero zeta potential) mit der Wirkung bakterieller Repulsion.

Nachfolgend wird die Erfindung auch hinsichtlich weiterer Merkmale und Vorteile anhand von Ausführungsbeispielen beschrieben, die anhand der Figur näher erläutert werden.

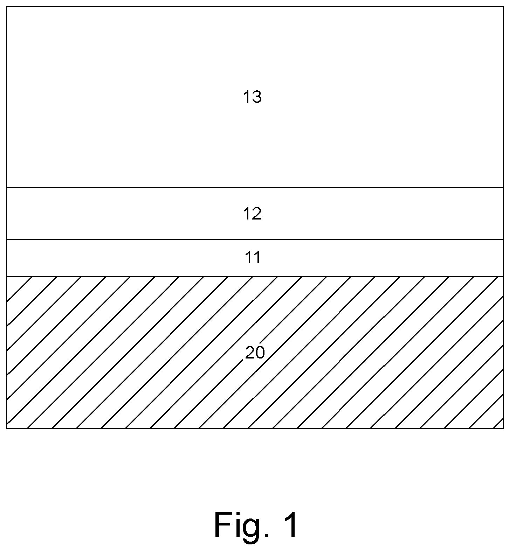

Hierbei zeigt Fig. 1 eine schematische Ansicht eines Elements mit einer erfindungsgemäßen Beschichtung.

Fig. 1 zeigt ein Element 20, das durch ein in Hart- und Weichgewebe integrierbares Implantat wie eine Schraube oder Platte gebildet ist. Auf dem Element 20 ist eine erfindungsgemäße Beschichtung aufgebracht, die aus mehreren Gradientenschichten 11, 12, 13 besteht. Die unterste, auf dem Element 20 ausgebildete Gradientenschicht 11 ist eine metallische Haftvermittlungsschicht. Die äußerste Gradientenschicht 13 ist eine weiße Schicht, die ein Metalloxid mit voller Stöchiometrie enthält. Zwischen den Gradientenschichten 11 und 13 sind eine oder mehrere Gradientenschichten 12 ausgebildet, deren Sauerstoffgehalt von der untersten, auf dem Element 20 ausgebildeten Gradientenschicht 11 bis zu der äußersten Gradientenschicht 13 mit voller Stöchiometrie anwächst.

Im einfachsten Fall wird die weiße Schicht als Zirkondioxid eingestellt. Zirkondioxid wird als vollkeramisches Material in der Implantologie eingesetzt. Bislang ist es jedoch noch nicht gelungen, Zirkondioxid als festhaftende weiße Schicht auf Titan-Implantatkörpern zu applizieren. In der erfindungsgemäßen Ausführung wird auf das Element 20, dessen Oberfläche vorzugsweise aufgerauht ist und in einer bevorzugten Ausführungsform aus Titan gebildet ist, zunächst eine metallische Schicht als unterste Gradientenschicht 11 aus Zirkonium in einer Schichtdicke von 20 nm abgeschieden. Im Folgenden wird mittels eines PVDtypischen reaktiven Prozess der Sauerstoff sukzessiv zugeführt und die Schicht über die Gradientenschichten 12 schließlich auf die volle Stöchiometrie ausgebildet, die in der äußersten Gradientenschicht 13 vorliegt. Die Gesamtschichtdicke der Beschichtung wird vorzugsweiße auf 5 Mikrometer eingestellt. Die Prozessführung kann so ausgeführt werden, dass die Abscheidung bei erhöhter Temperatur erfolgt, so dass nicht die sonst PVD-typische (röntgenamorphe) Schicht entsteht, sondern eine zumindest nanokristalline Schicht.

In einem zweiten Ausführungsbeispiel wird eine Mischphase mit 20 Mol% Nb₂O₅ und 80 Mol% Ta₂O₅ für die äußerste Gradientenschicht 13 eingestellt und die volle Stöchiometrie ausgehend von der untersten, metallischen Gradientenschicht 11 über die zwischenliegenden Gradientenschichten 12 aufgebaut. Die Schichten zeichnen sich durch eine besonders hohe biochemische Stabilität und ein negatives Oberflächenpotential aus. Das negative Oberflächenpotential bewirkt eine stabile Adsorption von Calciumionen und eine damit verbundene sichere Osteointegration.

Im weiteren Beispiel wird als äußerste Gradientenschicht 13 eine Schicht mit der Stöchiometrie ZrTi₂O₆ angewendet. (Ti,Zr)O₂₋ₓ Schichten sind hoch biokompatibel und blauschwarz aufgrund ihrer hohen Defektstruktur, ihrer röntgenamorphen Morphologie und nicht exakten Zusammensetzungseinstellung. Die erfindungsgemäße Beschichtung weist eine äußere Gradientenschicht 13 mit einer Bandlücke E_{g} von 3,1 eV oder größer auf und wird Stöchiometrie-genau eingestellt. Diese Gradientenschicht 13 ist nanokristallin. Aufgrund ihrer hohen negativen freien Bildungsenthalpie weist die äußerste Gradientenschicht 13 zusätzlich eine deutlich verbesserte biochemische Stabilität auf. Ihr point of zero potential liegt bei pH 6-7.

## Patentansprüche

1. Implantat, das durch ein Zahnimplantat mit einem enossalen Teil und einem Abutment gebildet ist,
**dadurch gekennzeichnet, dass**
eine weiße, bakterienresistente, biokompatible, haftfeste Beschichtung sowohl auf einen enossalen Teil als auch auf das Abutment des Zahnimplantats aufgebracht ist, wobei die Beschichtung einen Aufbau aus metallhaltigen Gradientenschichten (11, 12, 13) mit variierendem Sauerstoffgehalt aufweist, wobei die Bandlücke (E_{g}) der äußersten Gradientenschicht (13) größer als 3,1 eV ist, wobei die äußerste Gradientenschicht kristallin ist und wobei die Gradientenschichten (11, 12, 13) Tantal und/oder Niob aufweisen, wobei die äußerste Gradientenschicht eine titanfreie Mischphase aus 20 Mol-% Nb₂O₃ und 80 Mol-% Ta₂O₅ aufweist.

2. Implantat nach Anspruch 1, wobei die auf dem Implantat (20) aufgebrachte unterste Gradientenschicht (11) eine metallische Haftvermittlungsschicht ist, die äußerste Gradientenschicht (13) eine Metalloxidschicht mit voller Stöchiometrie ist, und wobei die zwischenliegenden Gradientenschichten (12) einen Sauerstoffgehalt aufweisen, der von der untersten, auf dem Implantat aufgebrachten Gradientenschicht (11) zu der äußersten Gradientenschicht (13) auf die volle Stöchiometrie anwächst.

3. Implantat nach Anspruch 1 oder 2, wobei mindestens eine der Gradientenschichten (12, 13) mit Sauerstoffgehalt, vorzugsweise mindestens die äußerste Gradientenschicht (13), Korngrößen von 5 nm oder größer aufweisen.

4. Implantat nach einem der vorhergehenden Ansprüche, wobei die Gradientenschichten (11, 12, 13) ferner Aluminium und/oder Zinn aufweisen.

5. Implantat nach einem der vorhergehenden Ansprüche, wobei die Konzentration der Metalle in den Gradientenschichten (12, 13) mit mindestens binären Oxiden so eingestellt ist, dass die Gradientenschichten (12, 13) mit mindestens binären Oxiden eine Bandlücke (E_{g}) größer als 3,1 eV aufweisen.

6. Implantat nach einem der vorhergehenden Ansprüche, wobei eine oder mehrere Gradientenschichten (11, 12, 13) Kohlenstoff und/oder Stickstoff und/oder Bor und/oder Fluor enthalten.

7. Implantat nach einem der vorhergehenden Ansprüche, wobei die unterste, auf dem Implantat aufgebrachte Gradientenschicht (11) eine Dicke von 50 nm oder weniger aufweist.

8. Implantat nach einem der vorhergehenden Ansprüche, wobei die Gesamtdicke der Gradientenschichten (12) mit verminderter Sauerstoffstöchiometrie 500 nm oder weniger, vorzugsweise 200 nm oder weniger, weiter vorzugsweise 100 nm oder weniger, weiter vorzugsweise 60 nm oder weniger beträgt.

9. Implantat nach einem der vorhergehenden Ansprüche, wobei die Dicke der äußersten Gradientenschicht (13) 10 µm oder weniger beträgt.

10. Implantat nach einem der vorhergehenden Ansprüche, wobei die Gesamtdicke der Beschichtung zwischen 3 µm und 7 µm, vorzugsweise zwischen 4 µm und 6 µm, weiter vorzugsweise zwischen 4,5 µm und 5,5 µm, beträgt.

11. Verfahren zur Herstellung einer weißen, bakterienresistenten, biokompatiblen, haftfesten Beschichtung auf einem Implantat nach einem der vorhergehenden Ansprüche, aufweisend die folgenden Schritte:
- Aufbringen einer metallischen Haftvermittlungsschicht als erste Gradientenschicht (11) auf die Oberfläche des Implantats (20) mittels PVD (physical vapor deposition)
- Aufbringen von Gradientenschichten (12, 13) aufweisend Tantal und/oder Niob sowie Sauerstoff, auf der metallischen Haftvermittlungsschicht (11) mit steigendem Sauerstoffgehalt durch Erhöhen des Sauerstoffgehalts bei Aufbringen der Gradientenschichten (12, 13) bis zum Erreichen der vollen Stöchiometrie in der äußersten Gradientenschicht (13),
wobei die Bandlücke (E_{g}) der äußersten Gradientenschicht (13) größer als 3,1 eV ist, wobei die äußerste Gradientenschicht eine titanfreie Mischphase aus 20 Mol-% Nb₂O₃ und 80 Mol-% Ta₂O₅ aufweist.

12. Verfahren nach Anspruch 11, wobei die Aufbringung der Gradientenschichten (11, 12, 13) bei einer Temperatur von 300°C oder höher erfolgt.

13. Verfahren nach Anspruch 11 oder 12, wobei die Gradientenschichten (11, 12, 13) unter Sauerstoffatmosphäre ausgeheilt werden.

14. Verfahren nach einem der Ansprüche 11 bis 13, wobei die Gradientenschichten (11, 12, 13) so ausgebildet werden, dass sie Korngrößen von 5 nm oder größer aufweisen.

15. Verfahren nach einem der Ansprüche 11 bis 14, wobei die Gradientenschichten (11, 12, 13) ferner Aluminium und/oder Zinn aufweisen.

16. Verfahren nach einem der Ansprüche 11 bis 15, wobei die Aufbringung der Gradientenschichten (11, 12, 13) derart erfolgt, dass die unterste, auf dem Implantat (20) aufgebrachte Gradientenschicht (11) eine metallische Haftvermittlungsschicht ist, die äußerste Gradientenschicht (13) eine Metalloxidschicht mit voller Stöchiometrie ist, und wobei die zwischenliegenden Gradientenschichten (12) einen Sauerstoffgehalt aufweisen, der von der untersten, auf dem Implantat aufgebrachten Gradientenschicht (11) zu der äußersten Gradientenschicht (13) auf die volle Stöchiometrie anwächst.

17. Verfahren nach einem der Ansprüche 11 bis 16, wobei die Konzentration der Metalle in den Gradientenschichten (12, 13) mit mindestens binären Oxiden so eingestellt ist, dass die Gradientenschichten (12, 13) mit mindestens binären Oxiden eine Bandlücke (E_{g}) größer als 3,1 eV aufweisen.

18. Verfahren nach einem der Ansprüche 11 bis 17, wobei eine oder mehrere Gradientenschichten (11, 12, 13) Kohlenstoff und/oder Stickstoff und/oder Bor und/oder Fluor enthalten.

## Claims

1. An implant which is formed by a dental implant having an enossal part and an abutment,
**characterised in that**
a white, bacteria-resistant, biocompatible, adherent coating is applied both onto an enossal part and onto the abutment of the dental implant, wherein the coating has a structure made from metalliferous gradient layers (11, 12, 13) having a varying oxygen content, wherein the band gap (E_{g}) of the outermost gradient layer (13) is greater than 3.1 eV, wherein the outermost gradient layer is crystalline, and wherein the gradient layers (11, 12, 13) have tantalum and/or niobium, wherein the outermost gradient layer has a titanium-free mixed phase made of 20 mol % of Nb₂O₃ and 80 mol % of Ta₂O₅.

2. The implant according to Claim 1, wherein the lowermost gradient layer (11) applied onto the implant (20) is a metallic adhesive bonding layer, the outermost gradient layer (13) is a metal oxide layer having full stoichiometry, and wherein the intermediate gradient layers (12) have an oxygen content which increases from the lowermost gradient layer (11) applied onto the implant to the outermost gradient layer (13) to the full stoichiometry.

3. The implant according to Claim 1 or 2, wherein at least one of the gradient layers (12, 13) having an oxygen content, preferably at least the outermost gradient layer (13), has grain sizes of 5 nm or greater.

4. The implant according to any one of the preceding claims, wherein the gradient layers (11, 12, 13) further have aluminium and/or tin.

5. The implant according to any one of the preceding claims, wherein the concentration of the metals in the gradient layers (12, 13) is adjusted with at least binary oxides so that the gradient layers (12, 13) having at least binary oxides have a band gap (E_{g}) of greater than 3.1 eV.

6. The implant according to any one of the preceding claims, wherein one or more gradient layers (11, 12, 13) contain(s) carbon and/or oxygen and/or boron and/or fluorine.

7. The implant according to any one of the preceding claims, wherein the lowermost gradient layer (11) applied onto the implant has a thickness of 50 nm or less.

8. The implant according to any one of the preceding claims, wherein the total thickness of the gradient layers (12) having a reduced oxygen stoichiometry is 500 nm or less, preferably 200 nm or less, further preferably 100 nm or less, further preferably 60 nm or less.

9. The implant according to any one of the preceding claims, wherein the thickness of the outermost gradient layer (13) is 10 µm or less.

10. The implant according to any one of the preceding claims, wherein the total thickness of the coating is between 3 µm and 7 µm, preferably between 4 µm and 6 µm, further preferably between 4.5 µm and 5.5 µm.

11. A method for producing a white, bacteria-resistant, biocompatible, adherent coating on an implant according to any one of the preceding claims, having the following steps:
- applying a metallic adhesive bonding layer as a first gradient layer (11) onto the surface of the implant (20) by means of PVD (physical vapour deposition),
- applying gradient layers (12, 13) having tantalum and/or niobium, as well as oxygen, onto the metallic adhesive bonding layer (11) having an increasing oxygen content by increasing the oxygen content during the application of the gradient layers (12, 13) until the full stoichiometry in the outermost gradient layer (13) is reached,
wherein the band gap (E_{g}) of the outermost gradient layer (13) is greater than 3.1 eV, wherein the outermost gradient layer has a titanium-free mixed phase made of 20 mol % of Nb₂O₃ and 80 mol % of Ta₂O₅.

12. The method according to Claim 11, wherein the application of the gradient layers (11, 12, 13) is carried out at a temperature of 300°C or higher.

13. The method according to Claim 11 or 12, wherein the gradient layers (11, 12, 13) are cured under an oxygen atmosphere.

14. The method according to any one of Claims 11 to 13, wherein the gradient layers (11, 12, 13) are configured so that they have grain sizes of 5 nm or greater.

15. The method according to any one of Claims 11 to 14, wherein the gradient layers (11, 12, 13) further have aluminium and/or tin.

16. The method according to any one of Claims 11 to 15, wherein the application of the gradient layers (11, 12, 13) is carried out such that the lowermost gradient layer (11) applied onto the implant (20) is a metallic adhesive bonding layer, the outermost gradient layer (13) is a metal oxide layer having full stoichiometry, and wherein the intermediate gradient layers (12) have an oxygen content which increases from the lowermost gradient layer (11) applied onto the implant to the outermost gradient layer (13) to the full stoichiometry.

17. The method according to any one of Claims 11 to 16, wherein the concentration of the metals in the gradient layers (12, 13) is adjusted with at least binary oxides so that the gradient layers (12, 13) having at least binary oxides have a band gap (E_{g}) of greater than 3.1 eV.

18. The method according to any one of Claims 11 to 17, wherein one or more gradient layers (11, 12, 13) contain(s) carbon and/or oxygen and/or boron and/or fluorine.

## Revendications

1. Implant, qui est formé par un implant dentaire comportant une partie intra-osseuse et un pilier,
**caractérisé en ce que**
un revêtement blanc, résistant aux bactéries, biocompatible, adhésif est appliqué aussi bien sur une partie intra-osseuse que sur le pilier de l'implant dentaire, le revêtement présentant une structure constituée par des couches de gradient (11, 12, 13) contenant du métal comportant une teneur variable en oxygène, la bande interdite (E_{g}) de la couche de gradient externe (13) étant supérieure à 3,1 eV, la couche de gradient externe étant cristalline et les couches de gradient (11, 12, 13) présentant du tantale et/ou du niobium, la couche de gradient externe présentant une phase mixte exempte de titane constituée par 20% en mole de Nb₂O₃ et par 80% en mole de Ta₂O₅.

2. Implant selon la revendication 1, la couche de gradient inférieure (11) appliquée sur l'implant (20) étant une couche métallique de promoteur d'adhérence, la couche de gradient externe (13) étant une couche d'oxyde de métal présentant une stœchiométrie complète et les couches de gradient intermédiaires (12) présentant une teneur en oxygène qui croît depuis la couche de gradient inférieure (11) appliquée sur l'implant vers la couche de gradient externe (13) jusqu'à la stœchiométrie complète.

3. Implant selon la revendication 1 ou 2, au moins l'une des couches de gradient (12, 13) comportant une teneur en oxygène, de préférence au moins la couche de gradient externe (13), présentant des grosseurs de grain de 5 nm ou plus.

4. Implant selon l'une des revendications précédentes, les couches de gradient (11, 12, 13) présentant en outre de l'aluminium et/ou de l'étain.

5. Implant selon l'une des revendications précédentes, la concentration en métaux dans les couches de gradient (12, 13) comportant des oxydes au moins binaires étant réglée de telle sorte que les couches de gradient (12, 13) comportant des oxydes au moins binaires présentent une bande interdite (E_{g}) supérieure à 3,1 eV.

6. Implant selon l'une des revendications précédentes, une ou plusieurs couches de gradient (11, 12, 13) contenant du carbone et/ou de l'azote et/ou du bore et/ou du fluor.

7. Implant selon l'une des revendications précédentes, la couche de gradient inférieure (11), appliquée sur l'implant, présentant une épaisseur de 50 nm ou moins.

8. Implant selon l'une des revendications précédentes, l'épaisseur totale des couches de gradient (12) présentant une stœchiométrie en oxygène réduite étant de 500 nm ou moins, de préférence de 200 nm ou moins, plus préférablement de 100 nm ou moins, encore plus préférablement de 60 nm ou moins.

9. Implant selon l'une des revendications précédentes, l'épaisseur de la couche de gradient externe (13) étant de 10 µm ou moins.

10. Implant selon l'une des revendications précédentes, l'épaisseur totale du revêtement étant située entre 3 µm et 7 µm, de préférence entre 4 µm et 6 µm, plus préférablement entre 4,5 µm et *5,5* µm.

11. Procédé pour la fabrication d'un revêtement blanc, résistant aux bactéries, biocompatible, adhésif sur un implant selon l'une des revendications précédentes, présentant les étapes suivantes :
- application d'une couche métallique de promoteur d'adhérence en tant que première couche de gradient (11) sur la surface de l'implant (20) au moyen de PVD (dépôt physique en phase vapeur),
- application de couches de gradient (12, 13), présentant du tantale et/ou du niobium ainsi que de l'oxygène, sur la couche métallique de promoteur d'adhérence (11) avec une teneur croissante en oxygène par augmentation de la teneur en oxygène lors de l'application des couches de gradient (12, 13) jusqu'à atteindre la stœchiométrie complète dans la couche de gradient externe (13),
la bande interdite (E_{g}) de la couche de gradient externe (13) étant supérieure à 3,1 eV, la couche de gradient externe présentant une phase mixte exempte de titane constituée par 20% en mole de Nb₂O₃ et par 80% en mole de Ta₂O₅.

12. Procédé selon la revendication 11, l'application des couches de gradient (11, 12, 13) ayant lieu à une température de 300°C ou plus.

13. Procédé selon la revendication 11 ou 12, les défauts des couches de gradient (11, 12, 13) étant réparés sous une atmosphère d'oxygène.

14. Procédé selon l'une des revendications 11 à 13, les couches de gradient (11, 12, 13) étant conçues de telle sorte qu'elles présentent des grosseurs de grain de 5 nm ou plus.

15. Procédé selon l'une des revendications 11 à 14, les couches de gradient (11, 12, 13) présentant en outre de l'aluminium et/ou de l'étain.

16. Procédé selon l'une des revendications 11 à 15, l'application des couches de gradient (11, 12, 13) ayant lieu de telle sorte que la couche de gradient inférieure (11) appliquée sur l'implant (20) est une couche métallique de promoteur d'adhérence, la couche de gradient externe (13) est une couche d'oxyde de métal présentant une stœchiométrie complète et les couches de gradient intermédiaires (12) présentent une teneur en oxygène qui croît depuis la couche de gradient inférieure (11) appliquée sur l'implant vers la couche de gradient externe (13) jusqu'à la stœchiométrie complète.

17. Procédé selon l'une des revendications 11 à 16, la concentration en métaux dans les couches de gradient (12, 13) comportant des oxydes au moins binaires étant réglée de telle sorte que les couches de gradient (12, 13) comportant des oxydes au moins binaires présentent une bande interdite (E_{g}) supérieure à 3,1 eV.

18. Procédé selon l'une des revendications 11 à 17, une ou plusieurs couches de gradient (11, 12, 13) contenant du carbone et/ou de l'azote et/ou du bore et/ou du fluor.
